# EUROPEAN PATENT SPECIFICATION

(11) **EP 1 989 543 B1**
(45) Date of publication and mention of the grant of the patent: **28.09.2016**
(21) Application number: 07703362.9
(22) Date of filing: 08.02.2007
(51) Int. Cl.: G01N 33/50, G01N 33/543, G01N 33/68, G01N 27/414

(54) **METHOD AND SYSTEM FOR DETECTING PHARMACOLOGICALLY ACTIVE SUBSTANCES BY MEASURING MEMBRANE CURRENTS WITH EXTRACELLULAR SENSORS**
VERFAHREN UND SYSTEM ZUM NACHWEIS PHARMAKOLOGISCH WIRKSAMER SUBSTANZEN DURCH MESSEN VON MEMBRANSTRÖMEN MIT EXTRAZELLULÄREN SENSOREN
PROCÉDÉ ET SYSTÈME DE DÉTECTION DE SUBSTANCES PHARMACOLOGIQUEMENT ACTIVES PAR MESURE DE COURANTS MEMBRANAIRES AU MOYEN DE CAPTEURS EXTRACELLULAIRES

(30) Priority: 10.02.2006 US 771876 P
(43) Date of publication of application: 12.11.2008
(73) Proprietor: Max-Planck-Gesellschaft zur Förderung der Wissenschaften e.V., 80539 München (DE)
(72) Inventor: PEITZ, Ingmar, 80686 München (DE); FROMHERZ, Peter, 80686 München (DE)
(74) Representative: Müller Hoffmann & Partner
(86) International application number: PCT/EP2007/001087
(87) International publication number: WO 2007/090649

(56) References cited:
- EP-A- 1 400 805
- WO-A2-2005/022113
- VOELKER MORITZ ET AL: "Signal transmission from individual mammalian nerve cell to field-effect transistor." SMALL (WEINHEIM AN DER BERGSTRASSE, GERMANY) FEB 2005, vol. 1, no. 2, February 2005 (2005-02), pages 206-210, XP002431370 ISSN: 1613-6829 cited in the application
- OZ MURAT ET AL: "Direct activation by dopamine of recombinant human 5-HT1A receptors: Comparison with human 5-HT2C and 5-HT3 receptors." SYNAPSE (HOBOKEN), vol. 50, no. 4, 15 December 2003 (2003-12-15), pages 303-313, XP002431371 ISSN: 0887-4476
- PEITZ, INGMAR; VÖLKER, MORITZ; FROMHERZ, PETER: "Serotonin-Gated Ion Channels on Transistor Chips for Cell-Based Biosensors in Proceedings of the annual fall meeting, German Society for Biochemistry and Molecular Biology (GBM), Berlin/Potsdam, Germany, September 18-21, 2005" [Online] 2005, WISSENSCHAFT ONLINE GMBH , XP002431372 Retrieved from the Internet: URL:http://www5.spektrum.de/gbm/fall05/hom epage/abstract_detail.php?artikel_id=1244> abstract
- HUANG J ET AL: "5-HT3A receptor subunits in the rat medial nucleus of the solitary tract: subcellular distribution and relation to the serotonin transporter" BRAIN RESEARCH, ELSEVIER, AMSTERDAM, NL, vol. 1028, no. 2, 3 December 2004 (2004-12-03), pages 156-169, XP004622471 ISSN: 0006-8993
- DATABASE MEDLINE [Online] US NATIONAL LIBRARY OF MEDICINE (NLM), BETHESDA, MD, US; 30 April 1999 (1999-04-30), RUDY B ET AL: "Contributions of Kv3 channels to neuronal excitability." XP002431374 Database accession no. NLM10414303

## Description

The present invention relates to a bioelectronic device comprising a living cell which is in operative contact with an extracellular planar potential-sensitive electrode, e.g. a field effect transistor. The cell comprises first and second ion channel/receptor systems which are responsive to stimuli. The ion channels are selected such that the ion flux of the first ion channel is directed against the ion flux of the second ion channel. Thus, the device is suitable as a bioelectronic sensor. Further, the invention relates to a method for determining the response of the cell to a stimulus. The method is e.g. suitable for drug screening.

To determine the pharmaceutical effect of test substances, often so-called cellular screening assays are performed in which a cell to be tested containing a receptor system is brought into contact with a test substance in order to examine its function as an effector on the cellular receptor system.

US Patent 6,602,399 discloses bioelectronic devices which combine receptor-effector systems with the functional characteristics of ion channels. The activity of these ion channels is modulated due to the effect of the receptor-effector system. This modulation can be detected by an extracellular planar potential sensitive electrode.

Voelker et al. (small 2005, 1, No. 2, 206-210) show that local transistor recording of individual mammalian neurons is possible with electrolyte-oxide-silicon (EOS) transistors having a sufficiently low noise level.

The study of Oz et al. (SYNAPSE 50: 303-313, 2003) analyses the effects of dopamine on the function of human 5-HT_{1A}, 5-HT_{2C} and 5-HT₃ expressed in *Xenopus* oocytes and CHO-K1 cells.

EP 1 400 805 A1 discloses a screening method on cells immobilized on electrodes which uses low salt media.

Peitz et al. (poster abstract, Proceedings of annual fall meeting, German Society for Biochemistry and Molecular Biology (GBM), Berlin/Potsdam, Germany, September 18-21, 2005) describe HEK293 cells expressing the serotonin receptor 5-HT3A cultured on field effect transistors.

WO 2005/022113 discloses exposing a population of cells to electric fields to set the transmembrane potential to a pre-selected voltage level for characterizing the biological activity of candidate compounds.

There is, however, still a need for devices and methods which allow a more efficient screening of many cells. This could simplify the procedure of pharmaceutical tests.

The present invention is described by claims 1-26.

A subject matter of the present invention is a bioelectronic device comprising
(a) an isolated single cell which is a living cell expressing (i) a first ion channel/receptor system wherein said first ion channel is a ligand-gated channel, and is responsive to a change in the characteristics of the first receptor and (ii) a second ion channel/receptor system wherein said second ion channel is responsive to a change in the characteristics of the second receptor,
   wherein the first ion channel and the second ion channel direct a countercurrent flux of ions with a same charge or a co-current flux of ions with a different charge, and
(b) an extracellular planar potential-sensitive electrode which is electrically insulated against the cell culture medium of the isolated cell, wherein the isolated cell is cultivated on said electrode and is in operative contact with said electrode, so that an electrical signal of the isolated cell can be measured,
wherein the isolated cell is transfected with a first nucleic acid molecule encoding components of the first ion channel/receptor system and with a second nucleic acid molecule encoding components of the second ion channel/receptor system.

The device of the present invention comprises an isolated living cell. This cell may be a microorganism, e.g. a bacterial cell or a yeast or fungal cell. Preferably, however, the cell is a eukaryotic cell, more preferably, a mammalian cell. Further, it is preferred that the cell overexpresses the ion channel/receptor systems, i.e. the cell is manipulated, e.g. by genetic engineering or mutation in a way that components of the ion channel/receptor systems are expressed in a higher amount than in a comparative untreated cell. The cell is transfected, e.g. stably transfected with nucleic acid molecules encoding components of the ion channel/receptor systems. In this embodiment of the invention the cell comprises heterologous nucleic molecules which encode at least a part of the components of the ion channel/receptor systems and which allow overexpression of said components.

The device of the present invention expresses at least a first ion channel/receptor system and at least a second ion channel/receptor system. By selecting the ion channels such that the ion flux of the first ion channel is directed against the ion flux of the second ion channel, a depolarization/repolarization of the cell membrane is effected resulting in an increased signal in response to a change in the characteristics of at least one receptor associated with one of the ion channels can be measured.

The first ion channel and the second ion channel are selected such that they direct a countercurrent flux of ions with a same charge, e.g. wherein the first ion channel directs an ion flux of a first ion species into the cell and the second ion channel directs an ion flux of a second species out of the cell, wherein the first and the second ion species have the same charge, i.e. both ion species have a positive charge or both ion species have a negative charge. The ions may be cations, e.g. sodium and/or potassium ions. Alternatively, the ions may be anions, e.g. chloride ions.

Alternatively, the first ion channel and the second ion channel are selected such that they direct a co-current flux of ions with a different charge, e.g. wherein the first ion channel directs an ion flux of a first ion species into the cell and the second ion channel directs an ion flux of a second ion species into the cell, wherein the first and the second ion species have a different charge, i.e. one species has a positive charge and the other species has a negative charge. In this embodiment, a cation species, e.g. sodium and/or potassium ions, may be combined with an anion species, e.g. chloride ions.

An ion channel/receptor system comprises a polypeptide or a plurality of polypeptides. On the one hand, an ion channel/receptor system comprises an ion channel component, e.g. a polypeptide or a plurality of polypeptides being capable of mediating an ion, i.e. cation and/or anion current through a cell membrane. On the other hand, an ion channel/receptor system comprises a receptor component which is responsive to stimuli. The receptor may be the ion channel or a part of the ion channel. The receptor, however, may be a molecule which is different from the ion channel, which is, however, in operative connection with the ion channel, e.g. a change in the functional and/or conformational state of the receptor results in a change of the functional state of the ion channel thus resulting in a detectable change of ion current through the cell membrane. The stimuli by which the receptor may be mediated are preferably selected from changes in the potential (inside or outside the cell), the presence or absence of effectors, e.g. ligands of the receptor, illumination, mechanical stimulation, stimulation by stimulation spots on the electrode or combinations thereof. For example, the binding of ligands to a receptor may cause the production of second messenger molecules which interact with the ion channel.

Preferred examples of ion channels are extracellular ligand-gated channels, e.g. serotonin receptors, such as 5-HT3, e.g 5-HT3A, nACh receptors, GABAA receptors, glycine receptors, P2X receptors, NMDA receptors, AMPA receptors and kainate receptors; intracellular ligand-gated channels like InsP3-gated channels, CNG channels and DAG-gated channels, e.g. coupled to a heterologous receptor system, and voltage-gated potassium channels like Kv1.1, Kv1.2, Kv1.3, Kv1.4 and all other Kv channels; and voltage-gated chloride channels such as CIC0, CIC1, CIC3. Preferred examples of receptor systems coupled to ion channels are G protein-coupled receptors (GPCR), receptor tyrosine kinases and T-cell receptors. Further receptor systems are metabotropic neurotransmitter receptors for serotonin, glutamate, acetylcholine and/or GABA. These receptor systems may be responsive to an extracellular ligand and produce a second messenger, which may act as a ligand to an intracellular ligand-gated ion channel.

The cell is cultivated on a planar potential-sensitive electrode. Methods of cultivating cells on planar potential-sensitive electrodes are disclosed e.g. in S. Vassanelli, P. Fromherz "Neurons from Rat Brain Coupled to Transistors" Appl. Phys. A 65, 85-88 (1997). By means of these cultivation cells are obtained, which grow on the potential-sensitive regions of the electrode resulting in an operative contact of the cell and the electrode.

When a cell is attached to the electrode surface, which may be oxidized silicon, other insulated semiconductors or metal, the cell membrane and the electrode surface are separated by a cleft which may be filled with an electrolyte. The electrode is preferably electrically insulated against the culture medium of the cell. Thus, a sandwich structure is formed of e.g. silicon, silicon dioxide, cleft, cell membrane and cell interior. The width of the cleft is usually in the range of about 10 to about 100 nm, e.g. about 50 nm.

The electrode may be integrated on, e.g. embedded in a chip. The chip may comprise further devices such as stimulating spots, transistors etc. Preferably the chip has at least one integrated field-effect transistor comprising at least one source and drain or an electrode as stimulating spot for applying voltages. The potential sensitive electrode, however, may also be a metal electrode which may be integrated on a chip. The chip may comprise a plurality of electrodes, e.g. field-effect transistors, for example at least 10, preferably at least 100, and more preferably, at least 1,000 electrodes on a single chip.

In the device of the present invention, the cell preferably has an integral membrane structure, i.e. there is no electrode, e.g. patch clamp, inserted into the membrane of the cell. This structural integrity leads to an increase in stability of the system, particularly allowing a plurality of identical or different measurement cycles without destroying the cells.

The bioelectronic device may comprise a single isolated cell or a plurality of isolated cells each in operative contact with an electrode. For example, the device may comprise at least 10, and preferably at least 100 cells on a single chip. The cells may be identical, i.e. they may contain identical first and second ion channel/receptor systems, or they may be different, i.e. they may contain different combinations of ion channel/receptor systems.

The functional characteristics of the first and second ion channels in the cell may include an opening of the channels in response to stimuli which will cause an ion current or flux to flow through participating channels. These ion currents will also flow in the region of operative contact between cell and electrode resulting in a detectable signal which can be measured. The detectable signal may be e.g. a voltage drop due to a junction resistance by the narrow cleft between cell and substrate or the change of the surface potential of the electrode due to diffuse ion concentration changes in the operative contact zone.

A change in functional characteristics e.g. conductivity of the ion channel changes the ion current and therefore the electrical signal detected by the electrode. Since the ion channels are responsive to the effector-receptor system, an alteration in the effector-receptor system will modulate the opening of the ion channels and thus result in a detectable signal.

Ion channels, particularly the gating characteristics thereof, can be modulated by different methods, e.g. by voltage modulation across the membrane (voltage-gated ion channels), by ligands acting on the intracellular and/or extracellular side of the channel (ligand-gated ion channels), by mechanical changes (mechanically-gated ion channels) or by combinations thereof.

Voltage-gated ion channels, i.e. ion channels which are voltage sensitive, will change their conductivity with the potential drop over the membrane (Vm = Vintra - Vextra). If the electrolyte, i.e. the culture medium in which the cell is grown, is grounded (Vextra = 0 mV) this potential drop equals the intracellular membrane (Vm = Vintra). This potential drop may be measured. In another embodiment, the conductivity of voltage-gated ion channels may be changed by voltage modulation due to an interaction with other ion channels, e.g. by means of an action potential. Vm is changed due to ion currents flowing into a cell through different ion channels. This co-operation of several ion channels influences the potential drop over the membrane leading in some cases to an action potential. Moreover, the potential difference between intracellular and extracellular side of the membrane may be modulated by using stimulation spots on the electrode.

A stimulation spot may be integrated next to the potential-sensitive electrode being in operative contact to the cell (Stett et al., Phys. Rev. E 55 (1997), 85). Thus, a device with the features of stimulation and recording may be built. A stimulation spot can, e.g. trigger an action potential which then will be recorded by the extracellular electrode.

Ligands can modulate ion channels preferably by two mechanisms, ionotropic and second messenger systems. In an ionotropic system the ligand molecules bind directly to the ion channels and alter their gating characteristics, e.g. intracellular Ca2+ shifts the gating curve of some K+ channels (DiChiara and Reinhard, J. Physiol. 489.2 (1995), 403). In second messenger systems the ligand molecules bind to a receptor which will first trigger some other molecules before the ion channel is influenced, e.g. many glutamate-second messenger systems.

According to the present invention, two ion channels are combined wherein the ion flux of the first ion channel is directed against the ion flux of the second ion channel. In a preferred embodiment, the first ion channel is a ligand-gated ion channel, e.g. an extracellular ligand-gated ion channel or an intracellular ligand-gated channel optionally coupled to a heterologous receptor system, and the second ion channel is a non-ligand-gated ion channel, preferably a voltage-gated ion channel. Preferred examples of extracellular ligand-gated ion channels are 5-HT3A, a cation channel which directs a cation flux into the cell or other extracellular ligand-gated channels, e.g. as described above. Preferred examples of voltage-gated ion channels are Kv1.3, a potassium ion channel which directs a potassium flux out of the call, further voltage-gated potassium channels which direct a potassium flux out of the cell or chloride channels, which direct a chloride flux into the cell.

Surprisingly it was found that an effective signal may be generated in a cell comprising two ion channels with ion fluxes directed against each other. Without wishing to be bound by theory, the inventors believe that the signal is generated by a depolarization/repolarization process resulting from the combined activity of both ion channels together with differences in opening times for an ion channel, e.g. a ligand-gated ion channel in the part of the cell membrane in contact with the electrode as compared to the part of the cell membrane not in contact with the electrode.

The signal is preferably a voltage signal, particularly a change in the junction voltage in the cleft between the cell and the electrode. The signal may be generated by inducing a response to one or both of the ion channel-receptor systems in the cell. A response may be generated, e.g. by adding an agonist (or a test compound assumed to be an agonist) of a first ion channel/receptor system, e.g. comprising a ligand-gated ion channel. Alternatively, an antagonist or a presumed antagonist of a ligand-gated ion channel may be tested. In a further embodiment, agonists or antagonists of voltage-gated ion channels may be tested. The tests may be carried out optionally in the presence of further relevant compounds, e.g. antagonists or agonists of the second ion channel/receptor system.

Further, disclosed herein is a cell transfected with (i) at least one first nucleic acid molecule encoding components of a first ion channel/receptor system wherein said first ion channel is responsive to a change in the characteristics of the first receptor, and (ii) at least one second nucleic acid molecule encoding components of a second ion channel/receptor system wherein said second ion channel is responsive to a change in the characteristics of the second receptor, and wherein the ion flux of the first ion channel is directed against the ion flux of the second ion channel.

The cell as disclosed herein is suitable as a component of a bioelectronic device as indicated above or as a component in any other device capable of determining ion currents, e.g. by using voltage-dependent dyes as disclosed in EP-A-1 553 396.

The cell as disclosed herein and bioelectronic device of the invention are suitable as a sensor which allows the determination of a change in an environmental parameter as a detectable signal, e.g. on the electrode of the device, and which is suitable as a scientific tool for studying the conformational and functional states of membrane proteins.

Particularly, the environmental parameter is an effector for the receptor component of the first and/or second ion channel/receptor system. More particularly, the system is used to determine whether a test substance is capable of activating or inhibiting the receptor component of the first and/or second ion channel/receptor system. The receptor component may be a pharmaceutically relevant target molecule. Thus, the present invention provides an in vitro method for determining the response of an isolated cell to a stimulus, said method comprising stimulating the isolated cell in a bioelectronic device of the present invention or stimulating an isolated cell, and determining the response to the stimulus, e.g. by contacting a test substance with the device or cell and determining a response of an ion channel/receptor system to the test substance. The response may be determined, for example, as an electric signal, e.g. a voltage signal, by the electrode of the bioelectronic device, or as an optical signal, e.g. by using a voltage-dependent dye.

In another embodiment, the bioelectronic device or the cell may be used as a sensor to determine the presence or the amount of a substance which acts as an effector to a receptor component in the cell.

Further, the invention shall be explained by the following figures and examples:
Fig. 1:
   A schematic picture of an ionoelectronic sensor with a cell on an open field-effect transistor. The cell comprises first and second ion channel/receptor systems. The first ion channel/receptor system directs an ion current into the cell and the second ion channel/receptor system directs an ion current out of the cell. A thin cleft of electrolyte separates the attached membrane from the silicon dioxide of the silicon chip. A signal, e.g chemical signal, in the solution opens receptor channels in the free and in the attached membrane. Ionic current flows in both directions through the free and attached membrane, driven by a suitable thermodynamic force. The resulting superposition of ionic and capacitive current through the attached membrane flows along the narrow cleft and gives rise there to a voltage drop.
   A measurable signal is generated by a depolarization/repolarization process resulting from the ion channel activity and by different opening times for channels in the narrow cleft between cell membrane and electrode compared to the channels in the free cell membrane. The resulting measurable change of extracellular voltage in the cleft plays the role of a gate voltage for the open field-effect transistor and modulates the electronic current from source (S) to drain (D) in the silicon chip.
**Fig. 2****:**
   Recording of the extracellular voltage signal V_{J} in the cell-chip junction probed with a Field Effect Transistor gate. The cell was transfected with the ligand-gated ion channel 5-HT3A, and the voltage-gated ion channel Kv1.3 and stimulated with serotonin (100 µM). The bar on top of the image indicates the onset and duration of serotonin stimulation.
**Fig. 3****:**
   **A** Recording of the extracellular voltage signal caused by the application of 100 µM serotonin (bar on top) to another doubly transfected cell as recorded with a FET.
   **B** Recording of the same cell during the application of tropisetron (first bar), a specific blocker of 5-HT3A. Tropisetron (1 µM) was present before and during the stimulation with serotonin (second bar). The signal was lost by inhibition of the current through the 5-HT3A channel.
**Figure 4****:**
   **A** FET recording of the extracellular voltage signal caused by the application of 100 µM serotonin (bar on top) to another doubly transfected cell.
   **B** Recording of the extracellular voltage of the same cell during application with the agonist CPBG (100 µM, bar on top). Both agonists of the 5-HT3A receptor triggered a biphasic voltage signal.
**Figure 5****:**
   **A** Biphasic extracellular voltage signal of another doubly transfected cell, induced by the application of 100 µM serotonin (bar on top) and recorded with a FET under the cell.
   **B** Recording of the same cell during the application of 100 µM serotonin (second bar) in the presence of 5 nM margatoxin (MgTx, first bar), a specific blocker of the Kv1.3-channel at this concentration. The signal was reduced by an incomplete inhibition of the current through the Kv1.3-channel.
**Figure 6****:**
   **A** FET recording of the extracellular voltage of a doubly transfected cell during serotonin stimulation (second bar) in the presence of 5 nM margatoxin (first bar).
   **B** Signal of the same cell, triggered with serotonin (bar on top) after removal of margatoxin. A biphasic voltage signal was recovered.

### Example

### 1. Materials and Methods

### 1.1 Cells and Plasmids

HEK293 cells were cultured in plastic dishes (Becton Dickinson, No. 353001), in Dulbecco's modified Eagle's medium (GIBCO, No. 21885-025), supplemented with 10% heat-inactivated fetal bovine serum. The cDNA of the human ligand-gated ion channel 5-HT3A was purchased from Invitrogen (Ultimate ORF clone collection, HORF01), and subcloned into the expression plasmid pcDNA3 (Invitrogen, No. V790-20), applying standard molecular biology methods. The construction of an expression plasmid for the voltage-gated potassium channel Kv1.3 was as described in J. Kupper: Functional expression of GFP-tagged Kv1.3 and Kv1.4 channels in HEK 293 cells. Eur. J. Neurosci. 10, 3908-12 (1998). The cells were stably transfected with one of the two expression plasmids using Geneticin (GIBCO, No. 11811-064), as the selection antibiotic (500 µg/ml). After generation of stable cell lines, the cells were transiently transfected with the other of the two expression plasmids together with the EGFP-C1 plasmid (BD Biosciences Clontech, No. 632317), which served as control for successful transfection. Transfections were done using the Effectene method (Qiagen, No. 301425), at 60-80% confluency.

### 1.2 Cell Culture on Silicon Chips

Silicon chips as described in M. Voelker, P. Fromherz: Signal transmission from individual mammalian nerve cell to field-effect transistor. Small 1, 206-210 (2005) with 128 Field Effect Transistors (FET) in two linear arrays and a culture chamber on top were cleaned, sterilized under UV-light and coated with 10 µg/ml fibronectin (Sigma, No. F2006). Cells were plated on these chips under normal culture conditions one day after transient transfection, and measurements were done one day after plating. Cells on transistor gates with the transiently expressed channels were identified by the green EGFP fluorescence.

### 1.3 Experimental conditions and solution exchange

During experiments the cells were flushed permanently with standard extracellular solution, consisting of (in mM): KCI 5.4, NaCl 135, CaCl₂ 1.8, MgCl₂ 1 Glucose 10, Hepes 5 (pH adjusted to 7.4 with NaOH). The ligands serotonin, CPBG (agonists of 5-HT3A) tropisetron and margatoxin (antagonists of 5-HT3A and Kv1.3, respectively), were obtained from Sigma (No. H9523, C144, T104, M8278), and dissolved in extracellular solution. Ligand solutions were applied rapidly (ms range), with a double barrelled glass pipette (Theta-Tube), connected to a piezoelectric actuator (Burleigh, LSS-3100). Different ligand solutions were directed to the glass pipette by an eight-channel valve control system (ALA, BPS-8).

### 2. Results

During application of ligands, the voltage in the cleft between transected cells and the chip surface was probed with the gates of Field Effect Transistors under the cells. Changes in this junction voltage were due to the ionic currents through both of the ion channels in the cell membrane, as was proven in the following way:

Cells expressing only the ligand-gated ion channel 5-HT3A failed to cause a voltage signal in the cell-chip junction upon stimulation with serotonin. Cells expressing only the voltage-gated channel Kv1.3 likewise failed to produce a signal upon stimulation with serotonin. On the other hand, the agonists serotonin and CPBG of the serotonin receptor 5-HT3A both caused a biphasic voltage signal in the transistor when applied to cells expressing both channels. The specific antagonist tropisetron of the 5-HT3A receptor blocked the signal as does an antagonist of the Kv1.3-channel, such as margatoxin, which is a specific blocker of Kv1.3 in the selected concentration range. Therefore the system is sensitive to ligands of the 5-HT3A receptor as well as to blockers of the Kv1.3-channel. The biphasic nature of the signal is caused by depolarization and subsequent repolarization of the cell membrane as well as by two different opening times for the 5-HT3A channels in the adhered cell membrane as compared to the channels in the free cell membrane.

Thus we provided a bioelectric device to screen for ligands to ligand-gated ion channels and to voltage-gated ion channels in a non-invasive and fast manner which could easily be expanded to a plurality of cells being processed in parallel. This would yield a high throughput screening device. Furthermore, this system is also applicable for screening ligands targeting voltage-gated channels as it is also sensitive to these molecules.

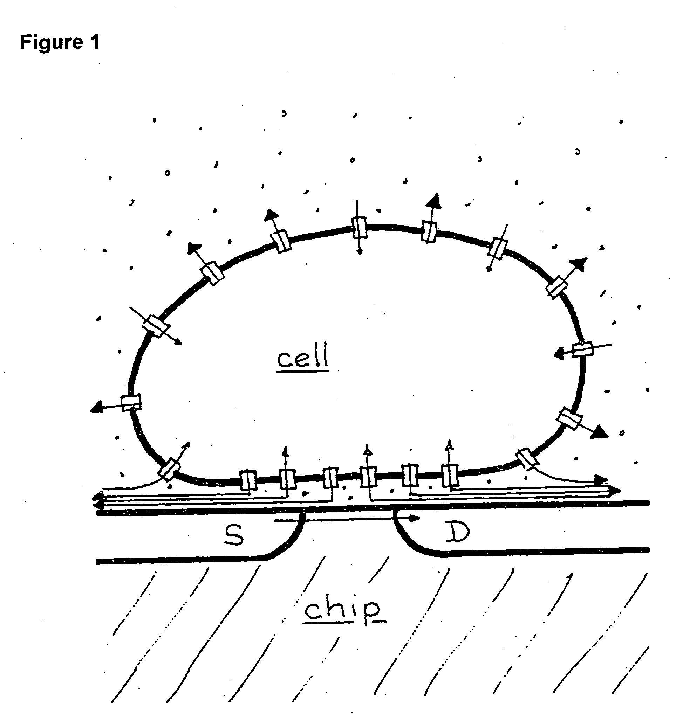

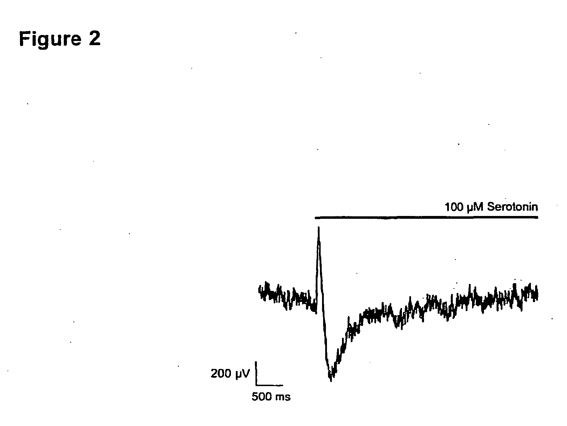

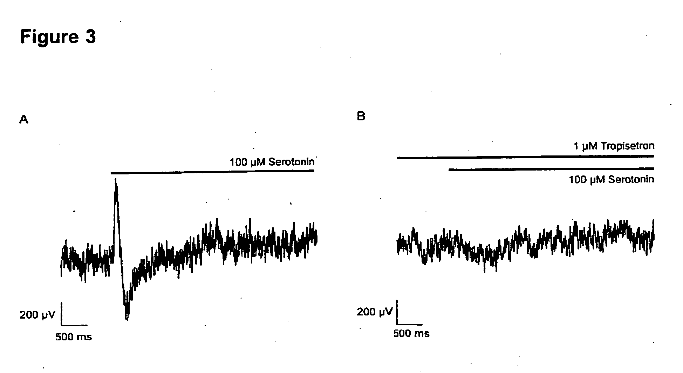

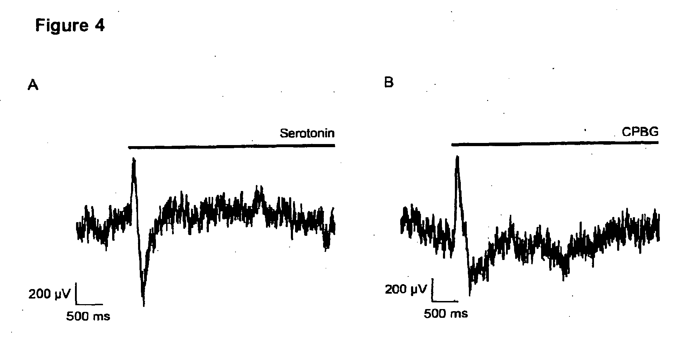

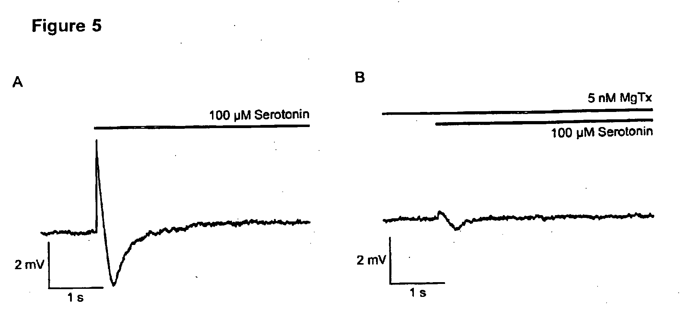

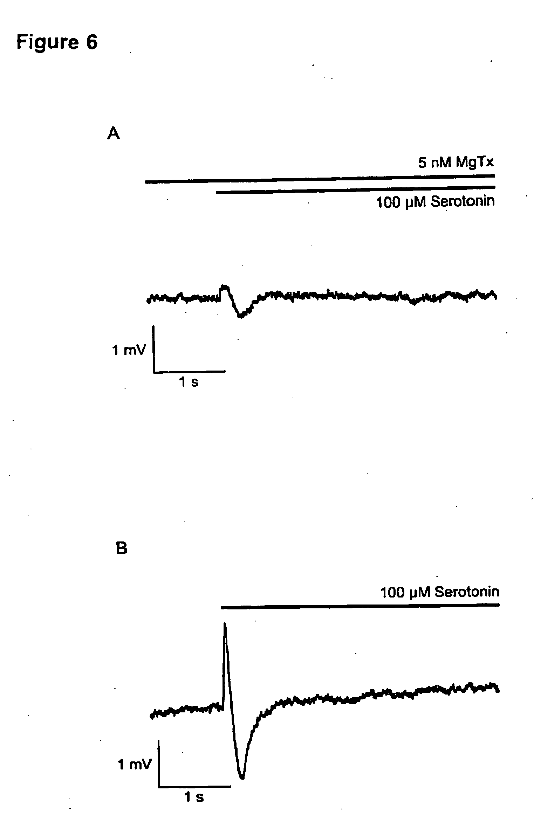

## Claims

1. A bioelectronic device comprising
(a) an isolated single cell which is a living cell expressing (i) a first ion channel/receptor system wherein said first ion channel is a ligand-gated channel, and is responsive to a change in the characteristics of the first receptor and (ii) a second ion channel/receptor system wherein said second ion channel is responsive to a change in the characteristics of the second receptor,
wherein the first ion channel and the second ion channel direct a countercurrent flux of ions with the same charge or a co-current flux of ions with a different charge, and
(b) an extracellular planar potential-sensitive electrode which is electrically insulated against the cell culture medium of the isolated cell, wherein the isolated cell is cultivated on said electrode and is in operative contact with said electrode, so that an electrical signal of the isolated cell can be measured,
wherein the isolated cell is transfected with a first nucleic acid molecule encoding components of the first ion channel/receptor system and with a second nucleic acid molecule encoding components of the second ion channel/receptor system.

2. The device of claim 1,
wherein said isolated cell is a eukaryotic cell.

3. The device of claim 1,
wherein the isolated cell overexpresses said first and/or second ion channel/receptor system.

4. The device of claim 1,
wherein said second ion channel is selected from ligand-gated channels and non-ligand gated channels.

5. The device of claim 4,
wherein the non-ligand-gated ion channel is a voltage-gated ion channel or a mechanically-gated ion channel.

6. The device of claim 5,
wherein the second channel is a voltage-gated channel.

7. The device of any one of claims 4 to 6,
wherein the first ion channel is an extracellular ligand-gated ion channel.

8. The device of any one of claims 4 to 6,
wherein the first ion channel is an intracellular ligand-gated ion channel optionally in combination with a heterologous receptor system.

9. The device of claim 1,
wherein the ions are cations.

10. The device of claim 1,
wherein the ions are anions.

11. The device of claim 1,
wherein the first ion channel is a ligand-gated cation channel, which directs a cation flux into the isolated cell or out of the isolated cell.

12. The device of claim 7,
wherein the first ion channel is selected from serotonin receptors.

13. The device of claim 8,
wherein the first ion channel is selected from InsP₃ channels, CNG channels, and DAG-gated channels.

14. The device of any one of claims 4 to 6,
wherein the second ion channel is a voltage-gated potassium or chloride channel, which directs a potassium or chloride flux out of the isolated cell or into the isolated cell.

15. The device of claim 14,
wherein the second ion channel is selected from Kv channels and CIC channels.

16. The device of claim 1,
wherein the isolated cell has an integral membrane structure.

17. The device of claim 1,
wherein the electrode is located on a chip.

18. The device of claim 1,
which comprises a plurality of electrodes.

19. The device of claim 1,
which comprises a plurality of isolated cells, which may be identical or different.

20. In vitro use of a bioelectronic device according to any one of claims 1 to 19 as a sensor.

21. The use of claim 20,
wherein a change in an environmental parameter is sensed as a detectable electrical or optical signal.

22. The use of claim 21,
wherein the environmental parameter is an effector for the receptor component of an ion channel/receptor system.

23. In vitro use of a bioelectronic device according to any one of claims 1-19 in a drug screening procedure.

24. The use of claim 23 for the determination whether a test substance is capable of activating or inhibiting a receptor component.

25. An in vitro method of determining the response of an isolated cell to a stimulus comprising stimulating the isolated cell in a device according to any one of claims 1-19, and determining the response to the stimulus.

26. The method of claim 25,
comprising contacting a test substance with the isolated cell and determining the response of an ion channel/receptor system to the test substance.

## Patentansprüche

1. Bioelektronische Vorrichtung, aufweisend:
(a) eine isolierte einzelne Zelle, welche eine lebende Zelle ist, exprimierend (i) ein erstes Ionenkanal/Rezeptorsystem, wobei der erste Ionenkanal ein liganden-gesteuerter Kanal ist und auf eine Änderung in den Charakteristiken des ersten Rezeptors anspricht, und (ii) ein zweites Ionenkanal/Rezeptorsystem, wobei der zweite Ionenkanal auf eine Änderung in den Charakteristiken des zweiten Rezeptors anspricht,
wobei der erste Ionenkanal und der zweite Ionenkanal einen Gegenstromfluss von Ionen mit der gleichen Ladung oder einen Gleichstromfluss von Ionen mit verschiedener Ladung leiten, und
(b) eine extrazelluläre planare potentialempfindliche Elektrode, welche gegen das Zellkulturmedium der isolierten Zelle elektrisch isoliert ist, wobei die isolierte Zelle auf der Elektrode kultiviert wird und mit der Elektrode in wirksamem Kontakt steht, so dass ein elektrisches Signal der isolierten Zelle gemessen werden kann,
wobei die isolierte Zelle mit einem ersten Nucleinsäuremolekül, das Komponenten des ersten Ionenkanal/Rezeptorsystems codiert, und mit einem zweitem Nucleinsäuremolekül transfiziert wird, das Komponenten des zweiten Ionenkanal/Rezeptorsystems codiert.

2. Vorrichtung nach Anspruch 1,
wobei die isolierte Zelle eine eukaryotische Zelle ist.

3. Vorrichtung nach Anspruch 1,
wobei die isolierte Zelle das erste und/oder zweite Ionenkanal/Rezeptorsystem überexprimiert.

4. Vorrichtung nach Anspruch 1,
wobei der zweite Ionenkanal aus liganden-gesteuerten Kanälen und nicht liganden-gesteuerten Kanälen ausgewählt ist.

5. Vorrichtung nach Anspruch 4,
wobei der nicht liganden-gesteuerte Ionenkanal ein spannungsgesteuerter Ionenkanal oder ein mechanisch gesteuerter Ionenkanal ist.

6. Vorrichtung nach Anspruch 5,
wobei der zweite Kanal ein spannungsgesteuerter Kanal ist.

7. Vorrichtung nach einem der Ansprüche 4 bis 6,
wobei der erste Ionenkanal ein extrazellulärer liganden-gesteuerter Ionenkanal ist.

8. Vorrichtung nach einem der Ansprüche 4 bis 6,
wobei der erste Ionenkanal ein intrazellulärer liganden-gesteuerter Ionenkanal wahlweise in Kombination mit einem heterologen Rezeptorsystem ist.

9. Vorrichtung nach Anspruch 1,
wobei die Ionen Kationen sind.

10. Vorrichtung nach Anspruch 1,
wobei die Ionen Anionen sind.

11. Vorrichtung nach Anspruch 1,
wobei der erste Ionenkanal ein liganden-gesteuerter Kationenkanal ist, welcher einen Kationenfluss in die isolierte Zelle oder aus der isolierten Zelle leitet.

12. Vorrichtung nach Anspruch 7,
wobei der erste Ionenkanal aus Serotoninrezeptoren ausgewählt ist.

13. Vorrichtung nach Anspruch 8,
wobei der erste Ionenkanal aus InsP₃-Kanälen, CNG-Kanälen und DAG-gesteuerten Kanälen ausgewählt ist.

14. Vorrichtung nach einem der Ansprüche 4 bis 6,
wobei der zweite Ionenkanal ein spannungsgesteuerter Kalium- oder Chloridkanal ist, welcher einen Kalium- oder Chloridfluss aus der isolierten Zelle oder in die isolierte Zelle leitet.

15. Vorrichtung nach Anspruch 14,
wobei der zweite Ionenkanal aus Kv-Kanälen und CIC-Kanälen ausgewählt ist.

16. Vorrichtung nach Anspruch 1,
wobei die isolierte Zelle eine integrale Membranstruktur aufweist.

17. Vorrichtung nach Anspruch 1,
wobei sich die Elektrode auf einem Chip befindet.

18. Vorrichtung nach Anspruch 1,
welche eine Vielzahl von Elektroden aufweist.

19. Vorrichtung nach Anspruch 1,
welche eine Vielzahl von isolierten Zellen aufweist, die identisch oder verschieden sein können.

20. In-vitro-Verwendung einer bioelektronischen Vorrichtung nach einem der Ansprüche 1 bis 19 als ein Sensor.

21. Verwendung nach Anspruch 20,
wobei eine Änderung in einem Umgebungsparameter als ein detektierbares elektrisches oder optisches Signal erfasst wird.

22. Verwendung nach Anspruch 21,
wobei der Umgebungsparameter ein Effektor für die Rezeptorkomponente eines Ionenkanal/Rezeptorsystems ist.

23. In-vitro-Verwendung einer bioelektronischen Vorrichtung nach einem der Ansprüche 1 bis 19 in einer Drugscreening- bzw. Wirkstoffscreening-Prozedur.

24. Verwendung nach Anspruch 23 für die Bestimmung, ob eine Testsubstanz eine Rezeptorkomponente aktivieren oder hemmen kann.

25. In-vitro-Verfahren zum Bestimmen der Antwort einer isolierten Zelle auf einen Stimulus, aufweisend ein Stimulieren der isolierten Zelle in einer Vorrichtung nach einem der Ansprüche 1 bis 19 und ein Bestimmen der Reaktion bzw. Antwort auf den Stimulus.

26. Verfahren nach Anspruch 25,
aufweisend ein Inkontaktbringen einer Testsubstanz mit der isolierten Zelle und ein Bestimmen der Antwort eines Ionenkanal/Rezeptorsystems auf die Testsubstanz.

## Revendications

1. Dispositif bioélectronique comprenant
(a) une cellule unique isolée qui est une cellule vivante exprimant (i) un système premier canal ionique/ récepteur dans lequel ledit premier canal ionique est un canal ligand-dépendant, et est réactif à un changement des caractéristiques du premier récepteur et (ii) un système second canal ionique/récepteur dans lequel ledit second canal ionique est réactif à un changement des caractéristiques du second récepteur,
dans lequel le premier canal ionique et le second canal ionique dirigent un flux à contre-courant des ions avec la même charge ou un flux à co-courant des ions avec une charge différente, et
(b) une électrode extracellulaire plane sensible au potentiel qui est électriquement isolée contre le milieu de culture cellulaire de la cellule isolée, dans lequel la cellule isolée est cultivée sur ladite électrode et est en contact fonctionnel avec ladite électrode, si bien qu'un signal électrique de la cellule isolée peut être mesuré,
dans lequel la cellule isolée est transfectée avec une première molécule d'acide nucléique codant pour les composants du système premier canal ionique/récepteur et avec une seconde molécule d'acide nucléique codant pour les composants du système second canal ionique/récepteur.

2. Dispositif selon la revendication 1,
dans lequel ladite cellule isolée est une cellule eucaryote.

3. Dispositif selon la revendication 1,
dans lequel la cellule isolée surexprime ledit système premier et/ou second canal ionique/récepteur.

4. Dispositif selon la revendication 1,
dans lequel ledit second canal ionique est choisi parmi des canaux ligand-dépendants et des canaux non ligand-dépendants.

5. Dispositif selon la revendication 4,
dans lequel le canal ionique non ligand-dépendant est un canal ionique dépendant du potentiel ou un canal ionique contrôlé mécaniquement.

6. Dispositif selon la revendication 5,
dans lequel le second canal est un canal dépendant du potentiel.

7. Dispositif selon l'une quelconque des revendications 4 à 6,
dans lequel le premier canal ionique est un canal ionique ligand-dépendant extracellulaire.

8. Dispositif selon l'une quelconque des revendications 4 à 6,
dans lequel le premier canal ionique est un canal ionique ligand-dépendant intracellulaire éventuellement en combinaison avec un système de récepteur hétérologue.

9. Dispositif selon la revendication 1,
dans lequel les ions sont des cations.

10. Dispositif selon la revendication 1,
dans lequel les ions sont des anions.

11. Dispositif selon la revendication 1,
dans lequel le premier canal ionique est un canal cationique ligand-dépendant, qui dirige un flux de cations dans la cellule isolée ou hors de la cellule isolée.

12. Dispositif selon la revendication 7,
dans lequel le premier canal ionique est choisi parmi des récepteurs de la sérotonine.

13. Dispositif selon la revendication 8,
dans lequel le premier canal ionique est choisi parmi des canaux InsP₃, des canaux CNG, et des canaux DAG-dépendants.

14. Dispositif selon l'une quelconque des revendications 4 à 6,
dans lequel le second canal ionique est un canal potassique ou chlorure dépendant du potentiel, qui dirige un flux de potassium ou de chlorure hors de la cellule isolée ou dans la cellule isolée.

15. Dispositif selon la revendication 14,
dans lequel le second canal ionique est choisi parmi des canaux Kv et des canaux CIC.

16. Dispositif selon la revendication 1,
dans lequel la cellule isolée a une structure de membrane intégrale.

17. Dispositif selon la revendication 1,
dans lequel l'électrode est localisée sur une puce.

18. Dispositif selon la revendication 1,
qui comprend une pluralité d'électrodes.

19. Dispositif selon la revendication 1,
qui comprend une pluralité de cellules isolées, qui peuvent être identiques ou différentes.

20. Utilisation *in vitro* d'un dispositif bioélectronique selon l'une quelconque des revendications 1 à 19 en tant que capteur.

21. Utilisation selon la revendication 20,
dans laquelle un changement d'un paramètre environnemental est capté comme un signal électrique ou optique détectable.

22. Utilisation selon la revendication 21,
dans laquelle le paramètre environnemental est un effecteur pour le composant récepteur d'un système canal ionique/récepteur.

23. Utilisation *in vitro* d'un dispositif bioélectronique selon l'une quelconque des revendications 1 à 19 dans une procédure de criblage de médicaments.

24. Utilisation selon la revendication 23 pour la détermination si une substance à tester est capable d'activer ou d'inhiber un composant récepteur.

25. Procédé *in vitro* de détermination de la réponse d'une cellule isolée à un stimulus comprenant la stimulation de la cellule isolée dans un dispositif selon l'une quelconque des revendications 1 à 19, et la détermination de la réponse au stimulus.

26. Procédé selon la revendication 25,
comprenant la mise en contact d'une substance à tester avec la cellule isolée et la détermination de la réponse d'un système canal ionique/récepteur à la substance à tester.
